(19) Europäisches Patentamt
European Patent Office
Office européen des brevets

(11) **EP 1 670 377 B1**

(12) **FASCICULE DE BREVET EUROPEEN**

(45) Date de publication et mention
de la délivrance du brevet:
**05.10.2016 Bulletin 2016/40**

(21) Numéro de dépôt: **04817203.5**

(22) Date de dépôt: **11.10.2004**

(51) Int Cl.:
*A61C 1/00* *(2006.01)*          *A61C 1/06* *(2006.01)*
*A61B 17/16* *(2006.01)*

(86) Numéro de dépôt international:
**PCT/EP2004/052497**

(87) Numéro de publication internationale:
**WO 2005/037124 (28.04.2005 Gazette 2005/17)**

(54) **DISPOSITIF CHIRURGICAL, NOTAMMENT DENTAIRE, COMPORTANT UN INSTRUMENT ET UN MODULE D'ASSERVISSEMENT**

CHIRURGISCHE VORRICHTUNG, INSBESONDERE DENTALVORRICHTUNG MIT EINEM SERVOKONTROLLINSTRUMENT UND EINEM MODUL

SURGICAL DEVICE, IN PARTICULAR A DENTAL DEVICE COMPRISING A SERVO-CONTROL INSTRUMENT AND A MODULE

(84) Etats contractants désignés:
**AT BE BG CH CY CZ DE DK EE ES FI FR GB GR HU IE IT LI LU MC NL PL PT RO SE SI SK TR**

(30) Priorité: **10.10.2003 CH 173403**

(43) Date de publication de la demande:
**21.06.2006 Bulletin 2006/25**

(73) Titulaire: **Dassym SA**
**2068 Hauterive (CH)**

(72) Inventeur: **VOILLAT, Jean-Pierre**
**CH-2857 Montavon (CH)**

(74) Mandataire: **P&TS SA (AG, Ltd.)**
**Av. J.-J. Rousseau 4**
**P.O. Box 2848**
**2001 Neuchâtel (CH)**

(56) Documents cités:
**EP-A- 0 254 215      EP-A- 0 688 539**
**WO-A-98/14129**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

La présente invention concerne un dispositif chirurgical, notamment dentaire, comportant un instrument, par exemple un instrument manuel, muni d'un moteur rotatif pour l'entraînement d'un outil et un module électronique d'asservissement de ce moteur.

De tels dispositifs sont fréquemment utilisés dans le domaine dentaire par exemple. Le dispositif comporte ou est constitué par une pièce à main qui peut être munie par exemple d'une fraise, d'un autre outil tournant, ou d'un dispositif entraîné par un moteur tournant. Le module électronique d'asservissement est souvent lié à la chaise du patient et relié à l'instrument manuel par un raccord flexible contenant des fils électriques et des tubes de passage pour l'air et l'eau.

Les moteurs rotatifs utilisés dans ce type de pièce à main sont de plus en plus souvent des moteurs sans collecteurs et sans balais qui ont l'avantage d'être plus robustes et de pouvoir être stérilisés complètement. Pour éviter un échauffement excessif de l'instrument, qui peut être amené à tourner à des vitesses importantes, par exemple jusqu'à 40'000 tours par minute, des moteurs triphasés sont préférables.

De tels dispositifs dentaires sont décrits par exemple dans les documents EP688539, US5543695, EP1228737, EP1302173, WO0105023, WO01/45248, EP1109301 ou WO0004631, auxquels le lecteur se référera utilement.

Le document WO9814129 décrit un dispositif chirurgical selon le préambule de la revendication 1.

Il est important que l'utilisateur de ce type de dispositif puisse contrôler avec précision et sur une large plage la vitesse de rotation de l'instrument. Un contrôle du couple est aussi nécessaire par exemple pour arrêter la rotation avant que l'outil ne casse. On connaît donc dans l'art antérieur des solutions dans lesquelles les instruments manuels sont munis de capteurs permettant de détecter en tout temps la position angulaire ou la vitesse du rotor du moteur. Le moteur est asservi avec des valeurs de consigne déterminées en tenant compte des signaux de mesure fournis par ces capteurs.

Afin d'augmenter la flexibilité du raccord entre le module d'asservissement et l'instrument, il est nécessaire de limiter le nombre de fils électriques passant à travers ce raccord. Les raccords disponibles commercialement et acceptés par les utilisateurs comportent donc deux, ou au plus quatre fils électriques.

Dans le cas d'un moteur triphasé, trois de ces fils sont utilisés pour l'alimentation des enroulements statoriques (phases du moteur). Le quatrième fil est souvent utilisé pour contrôler la lampe ou un autre accessoire associé à la pièce à main. Aucun conducteur électrique n'est donc disponible pour transmettre les signaux de mesure fournis par le capteur au module électronique d'asservissement.

Différentes solutions existent dans l'art antérieur dans lesquelles l'électronique d'asservissement est entièrement embarquée dans l'instrument manuel. Ce type de pièces à main est toutefois volumineux, lourd et donc difficile à manipuler. Par ailleurs, le coût d'un jeu de plusieurs instruments incluant tous une électronique d'asservissement devient prohibitif.

D'autres solutions ont été suggérées dans lesquels le module d'asservissement injecte sur au moins un des conducteurs de phase un signal haute fréquence déformé par les champs magnétiques statoriques et rotoriques. La mesure de cette déformation sur les conducteurs de retour permet de déterminer la position angulaire du rotor. Ce procédé est toutefois peu robuste et ne permet pas d'effectuer des mesures fiables et précises.

D'autres solutions d'asservissement utilisées emploient des raccords non standards avec plus de quatre fils électriques.

D'autres solutions encore nécessitent de modifier la configuration du rotor, par exemple sa forme ou la disposition d'aimants, pour permettre une mesure sans électronique embarquée. Ces solutions ne sont pas applicables avec des moteurs standards du commerce, et posent des contraintes supplémentaires importantes lors de la fabrication du moteur.

Un but de la présente invention est donc de proposer un instrument amélioré qui permet de résoudre les problèmes de l'art antérieur mentionnés.

Un but de la présente invention est en particulier de proposer un dispositif amélioré dans lequel la position angulaire du rotor peut être déterminée avec une précision et une résolution de l'ordre de 1 degré, et ce sur une très large plage de vitesse, par exemple de 1 à 40'000 tours par minute.

Un autre but est d'augmenter les possibilités de commande, de mesure et d'asservissement d'un instrument de ce type.

Selon l'invention, ces buts sont atteints au moyen d'un dispositif comprenant les caractéristiques de la revendication 1.

En particulier, ces buts sont atteints au moyen d'un dispositif chirurgical, notamment dentaire, comportant:

un instrument muni d'un moteur rotatif pour l'entraînement d'un outil et d'au moins un capteur pour déterminer au moins un paramètre de fonctionnement du moteur,
un module électronique d'asservissement du moteur,

un raccord flexible pour relier électriquement l'instrument avec le module électronique,

dans lequel l'instrument comprend un circuit électronique numérique pour traiter les signaux de sortie du capteur et pour les transmettre au module électronique au travers du raccord flexible.

Le traitement numérique des données de mesure fournies par le ou les capteurs permet de réduire considérablement la quantité d'informations devant être transmises au module d'asservissement. Par exemple, au lieu de fournir au module électronique des signaux analogiques bruts de sortie du ou des capteurs, le circuit électronique numérique peut délivrer à intervalles périodiques des mots numériques indiquant la position angulaire du rotor. Ces mots peuvent être transmis par exemple en série à l'aide d'un procédé de modulation numérique sur l'un des conducteurs de phase du moteur, ou sur le quatrième fil disponible.

Dans l'autre sens, le circuit électronique numérique et l'instrument peuvent être commandés par le module électronique au moyen de messages de commande numériques. Il est ainsi possible de commander de façon très souple le fonctionnement du ou des capteurs, du moteur, du circuit électronique numérique et d'éventuels circuits auxiliaires.

Une souplesse de mesure et de commande optimale peut ainsi être atteinte grâce à ce dialogue numérique bidirectionnel entre le module électronique d'asservissement et l'instrument. Le traitement numérique des données de mesure permet également de réduire les erreurs de mesure provoquées par des perturbations électrostatiques ou électromagnétiques lors de la transmission.

Le circuit électronique numérique dans l'instrument peut aussi traiter et transmettre des informations fournies par d'autres capteurs ou dispositifs de l'instrument, ou liés à l'instrument, par exemple des capteurs de couple, de température, des interrupteurs, des détecteurs de durée d'utilisation de l'ampoule, des détecteurs de type d'outil, etc.

La présente invention sera mieux comprise à la lecture de la description d'une version préférentielle illustrée par les figures, où:

la figure 1 représente un moteur sans balais incluant un rotor et trois bobinages,

la figure 2 représente des signaux électriques en cosinus et en sinus générés par le mouvement du rotor du moteur de la figure 1,

la figure 3 illustre l'extraction des tangentes de la position angulaire du moteur de la figure 1,

la figure 4 illustre la droite d'acquisition de position (DAP),

la figure 5 représente schématiquement le dispositif d'acquisition de la position angulaire du rotor du moteur de la figure 1 selon une version de l'invention,

la figure 6 montre un schéma synoptique de l'électronique embarquée dans l'instrument selon une version de l'invention,

les figures 7a, 7b, 7c et 7d représentent les signaux électriques transmis entre le module électronique d'asservissement et le moteur selon une version de l'invention,

la figure 8 représente un signal d'horloge et un signal combiné horloge/données correspondant selon une version de l'invention,

la figure 9 représente un message selon une version de l'invention,

la figure 10 illustre l'attribution des bits des messages envoyés par le microcontrôleur selon une version de l'invention,

la figure 11 illustre la structure d'un message auxiliaire selon une version de l'invention,

la figure 12a est un schéma de principe des drivers selon une version de l'invention,

la figure 12b illustre un moteur triphasé commandé par trois drivers,

la figure 13 illustre schématiquement un principe de base de mise en rotation du moteur,

la figure 14 montre différentes positions géométriques des capteurs de Hall,

la figure 15 illustre un processus de compensation de la position géométrique des capteurs de Hall,

la figure 16 illustre un dispositif de compensation de l'angle de réactualisation,

la figure 17 montre l'ensemble des dispositifs de compensation selon une version préférentielle de l'invention,

la figure 18 montre un dispositif chirurgical selon une version préférentielle de l'invention,

la figure 19 est une vue explosée de quelques éléments internes du moteur du dispositif de la figure 18.

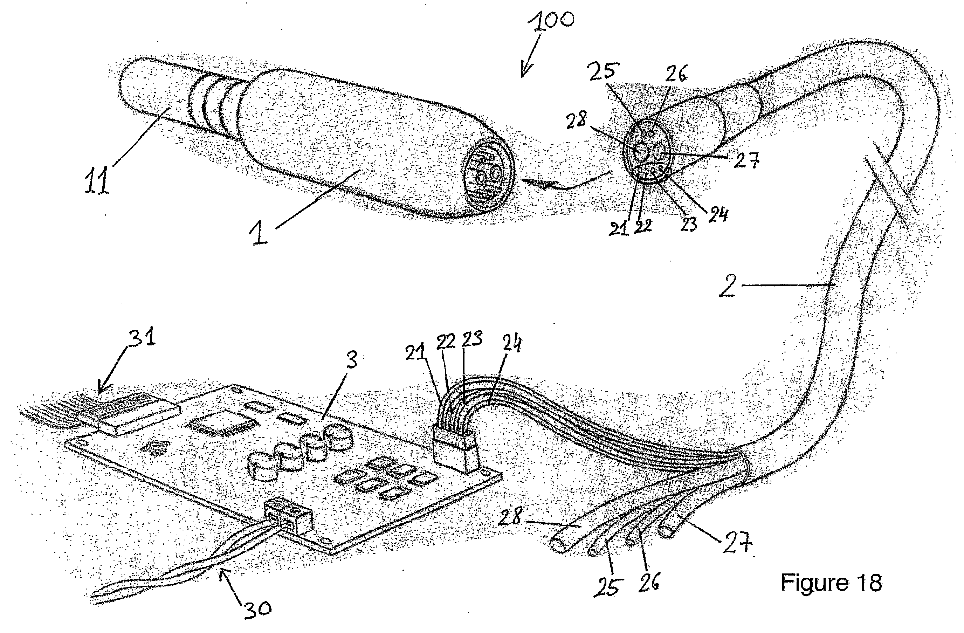

Le dispositif chirurgical de l'invention est de préférence un instrument manuel et comporte par exemple un moteur dans son boîtier ainsi qu'une pièce à main portant un outil entraîné par le moteur. Un tel dispositif est par exemple partiellement illustré à la figure 18. Le dispositif comprend un instrument 100 dont le moteur 1 est représenté. Le moteur 1 comporte de préférence un nez ou accrochage 11 destiné à recevoir par exemple une pièce à main non représentée. L'instrument 100 comprend ainsi le moteur 1 et par exemple la pièce à main. L'invention s'applique cependant aussi à des dispositifs dans lesquels le moteur, la pièce à main et éventuellement même l'outil forment une seule pièce.

L'instrument 100 est de préférence relié à un module d'asservissement 3 par un raccord flexible, par exemple un tuyau flexible 2 attaché au moteur 1. L'instrument 100 est alimenté par exemple en eau, en air et en électricité par le tuyau 2. Dans le cas d'un dispositif dentaire, le tuyau 2 comprend par exemple quatre contacts électriques 21, 22, 23, 24, et des tuyaux 25, 26, 27, 28 pour le passage d'air et/ou de liquide. Les tuyaux pour le passage d'air et/ou de liquide comprennent de préférence une conduite d'amenée d'eau 25 pour un spray, une conduite d'amenée d'air 26 également pour le spray, une conduite d'amenée d'air de refroidissement 27 destiné au refroidissement du moteur 1 et une conduite de retour d'air 28.

Le tuyau 2 est relié à son autre extrémité à une ou plusieurs sources d'air et d'eau non représentées ainsi que de préférence au module électronique d'asservissement 3 destiné en particulier à la gestion de l'alimentation électrique du moteur 1. Les contacts électriques 21, 22, 23, 24 du tuyau 2 sont ainsi de préférence connectés au module électronique d'asservissement 3. Dans le cas d'un moteur triphasé, trois des quatre fils électriques sont par exemple utilisés pour alimenter électriquement trois enroulements statoriques du moteur 1, et le quatrième fil électrique 24 est par exemple relié électriquement à un accessoire électrique de l'instrument 100 ou de la pièce à main de l'instrument, par exemple à une lampe permettant d'éclairer la zone de travail de l'instrument.

Le module électronique d'asservissement 3 est par ailleurs connecté, par exemple au moyen d'un câble électrique 30, à une source d'alimentation électrique non représentée, par exemple à une source de tension continue fournissant par exemple une tension de 34 volts. Le module électronique d'asservissement 3 est de préférence également connecté à un bus de commande 31 au travers duquel il peut par exemple communiquer avec un calculateur non représenté, un tableau de commande, etc.

## ACQUISITION DE LA POSITION DU MOTEUR

### Les éléments de l'acquisition

Selon un mode de réalisation de l'invention, l'instrument comprend des moyens d'acquisition de la position angulaire du rotor du moteur 1.

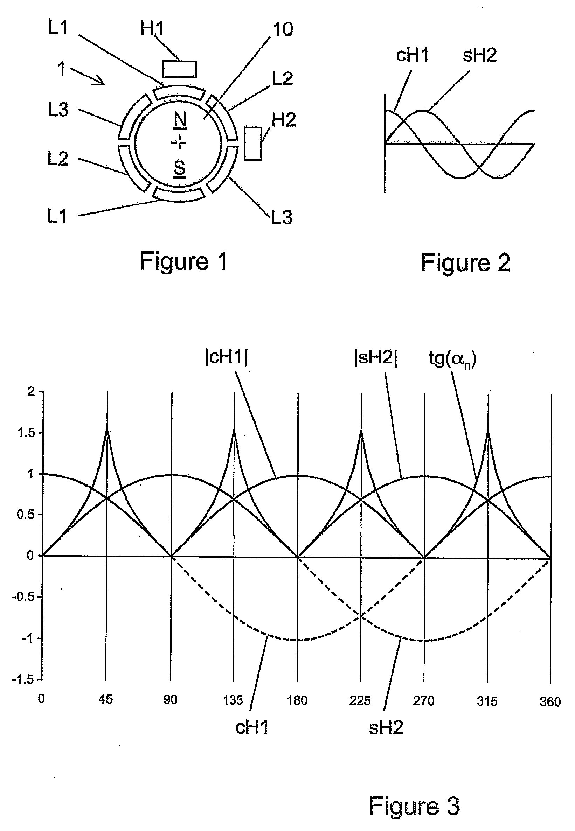

La figure 1 illustre schématiquement le moteur 1 d'un instrument selon une version préférentielle de l'invention. Le moteur 1 est par exemple un moteur sans balais incluant un rotor 10 et trois bobinages L1, L2, L3. Il s'agit ainsi par exemple d'un moteur sans collecteur triphasé comportant trois enroulements statoriques L1, L2, L3 montés en étoile. Le moteur 1 comprend en outre deux capteurs H1, H2 permettant de déterminer au moins un paramètre de fonctionnement du moteur. Les capteurs H1, H2 sont par exemple des capteurs analogiques à effet de Hall ou magnétorésistifs permettant de déterminer la position angulaire $\alpha$ instantanée du rotor 10. Ils sont disposés par exemple entre le rotor 10 et le stator L1, L2, L3 ou à l'extérieur du stator 10. Les deux capteurs H1, H2 sont de préférence disposés de manière à former entre eux un angle de 90°.

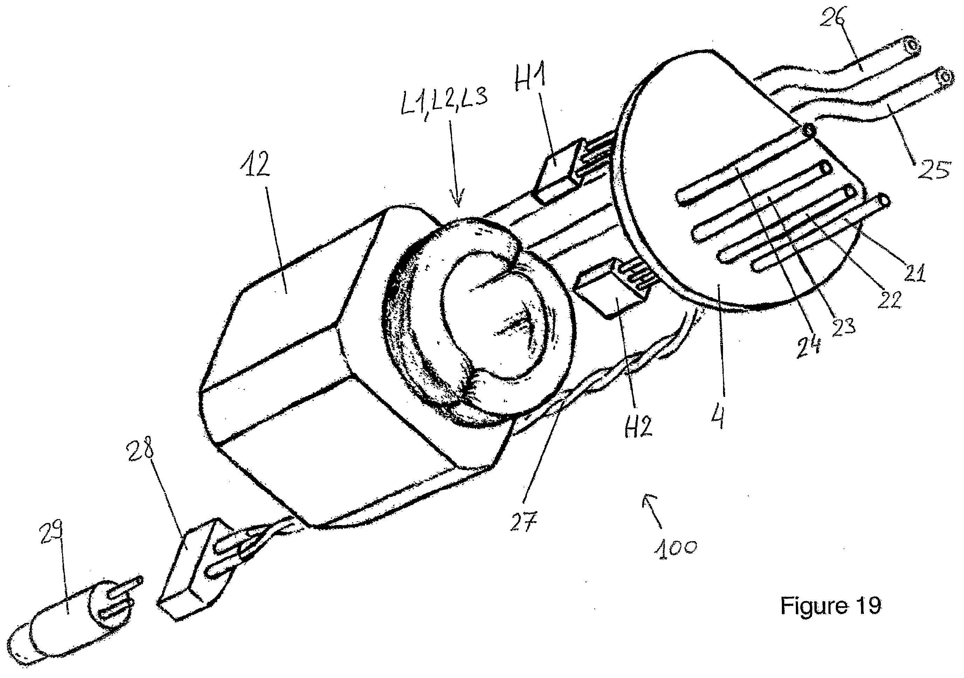

La figure 19 est une vue explosée de quelques éléments internes de l'instrument 100 du dispositif chirurgical selon une version préférentielle de l'invention. Sans indication contraire, les mêmes références désignent les mêmes éléments sur toutes les figures.

Selon une version préférentielle de l'invention, l'instrument 100 comprend un circuit électronique numérique 4 embarqué comprenant de préférence un microcontrôleur non représenté. La référence 12 désigne la culasse statorique du moteur. Un blindage non représenté est de préférence prévu entre les enroulements statoriques L1, L2, L3 du moteur 1 et le circuit électronique numérique 4.

Les contacts électriques 21, 22, 23, 24 sont de préférence en contact direct avec le circuit électronique numérique 4 auquel sont également connectés les capteurs H1, H2. Le circuit électronique numérique 4 est de préférence monté sur une carte de circuit imprimée perpendiculaire à l'axe de rotation du moteur. Les capteurs H1, H2 sont alors par exemple directement montés sur la carte de circuit imprimé.

Lorsque l'instrument 100 est monté, les capteurs H1, H2 sont par exemple logés dans un évidement dans les tôles statoriques du moteur 1 et/ou dans un évidement entre le rotor 10 et le stator.

L'instrument 100 comprend en outre par exemple un accessoire électrique. L'accessoire électrique est de préférence une ampoule 29, par exemple une LED, permettant d'éclairer la zone de travail. L'ampoule 29 est par exemple maintenue dans une prise 28 alimentée par un câble d'alimentation électrique 27. Une des bornes de l'accessoire électrique est par exemple reliée à un des fils 24 dédié à cet usage, à un des conducteurs de phase 21, 22, 23 du moteur et/ou au point neutre entre les phases du moteur. Une des bornes de l'accessoire électrique peut également être reliée au circuit électronique numérique 4. Le fonctionnement de l'accessoire électrique est alors par exemple commandé par le circuit électronique numérique 4.

La rotation du rotor 10 génère respectivement sur les capteurs H1 et H2 un signal électrique en cosinus cH1 et en sinus sH2 représentés à la figure 2. De préférence, les signaux cH1 et sH2 sont proportionnels respectivement au cosinus et au sinus de l'angle $\alpha$ ou position angulaire $\alpha$ du rotor 1.

Il est possible d'extraire la tangente de l'angle $\alpha$ ou position angulaire $\alpha$ du rotor 1 en effectuant:

$$tg(\alpha)=sH2 / cH1.$$

### Extraction de la tangente de l'angle de position

Malheureusement, la tangente tend vers infini. Pour éviter cet inconvénient, toute extraction de la tangente est de préférence ramenée dans la zone 0° - 45°, puis replacé dans sa zone d'origine.

Selon une version préférentielle de l'invention, une règle d'extraction est établie selon les conditions énoncées dans le tableau ci-dessous:

| | | | | | |
|---|---|---|---|---|---|
| 0°-45° | $cH1 \geq 0$ | $sH2 \geq 0$ | $|cH1| \geq |sH2|$ | $tg\alpha_1 = tg(0°) + tg[|sH2|/|cH1|]$ | $\alpha=0°+\alpha_0$ |
| 45°-90° | | | $|sH2| \geq |cH1|$ | $tg\alpha_0 = tg(45°) - tg[|cH1|/|sH2|]$ | $\alpha=90°-\alpha_1$ |
| 90°-135° | $cH1 < 0$ | | $|sH2| \geq |cH1|$ | $tg\alpha_2 = tg(0°) + tg[|cH1|/|sH2|]$ | $\alpha=90°+\alpha_2$ |
| 135°-180° | | | $|cH1| \geq |sH2|$ | $tg\alpha_3 = tg(45°) - tg[|sH2|/|cH1|]$ | $\alpha=180°-\alpha_3$ |
| 180°-225° | | $sH2 < 0$ | $|cH1| \geq |sH2|$ | $tg\alpha_5 = tg(0°) + tg[|sH2|/|cH1|]$ | $\alpha=180°+\alpha_4$ |
| 225°-270° | | | $|sH2| \geq |cH1|$ | $tg\alpha_4 = tg(45°) - tg[|cH1|/|sH2|]$ | $\alpha=270°-\alpha_5$ |
| 270°-315° | $cH1 \geq 0$ | | $|sH2| \geq |cH1|$ | $tg\alpha_6 = tg(0°) + tg[|cH1|/|sH2|]$ | $\alpha=270°+\alpha_6$ |
| 315-0° | | | $|cH1| \geq |sH2|$ | $tg\alpha_7 = tg(45°) - tg[|sH2|/|cH1|]$ | $\alpha=360°-\alpha_7$ |

La première colonne du tableau détermine des zones pour la valeur de la position angulaire $\alpha$. Les deuxième, troisième et quatrième colonnes indiquent les conditions remplies par les signaux en cosinus cH1 et en sinus sH2 ainsi que par leurs valeurs absolues |cH1|, resp. |sH2| dans chacune de ces zones. La cinquième colonne du tableau indique la formule permettant pour chaque zone de calculer la tangente de la position angulaire $\alpha_n$ (n=0-7) du rotor ramenée dans la zone angulaire 0°-45°. La position angulaire $\alpha$ du rotor est ensuite calculée pour chaque zone selon la formule exprimée dans la dernière colonne du tableau.

La figure 3 montre l'extraction des tangentes $\alpha_n$ (n=0-7). Elle illustre en particulier les signaux en cosinus cH1 et en sinus sH2, les valeurs absolues |cH1|, resp. |sH2| de ces signaux, ainsi que la tangentes $tg(\alpha_n)$ de la position angulaire $\alpha_n$ (n=0-7) du rotor ramenée dans la zone angulaire 0° - 45°.

### Détermination de la position angulaire

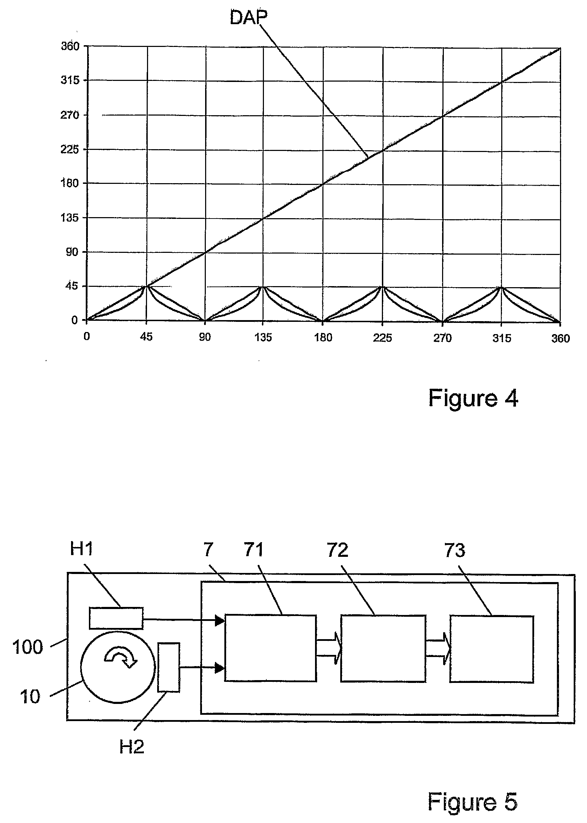

La figure 4 illustre la détermination de la position angulaire du rotor du moteur du dispositif de l'invention. La figure montre l'angle $\alpha_n$ (n=1,...,7) dont le calcul de la tangente est indiqué dans le tableau ci-dessus et dont la valeur varie entre 0° et 45°. La position angulaire $\alpha$ du rotor du moteur est représentée par la droite d'acquisition de position DAP. Elle est calculée à partir de la valeur de $\alpha_n$ selon la formule donnée pour chaque zone angulaire dans la dernière colonne du tableau.

L'acquisition de la position angulaire $\alpha$ du rotor 10 du moteur 1 est de préférence directement calculée selon la méthode expliquée ci-dessus par le circuit électronique numérique embarqué de l'instrument. Le circuit électronique numérique embarqué dans l'instrument 100 comprend de préférence un microcontrôleur embarqué 7 effectuant l'acquisition de position angulaire $\alpha$ du rotor 10 du moteur 1 selon le principe illustré à la figure 5. Les signaux analogiques générés par les capteurs H1, H2 lors de la rotation du rotor 10 du moteur de l'instrument sont envoyés au microcontrôleur 7 qui les convertit en signaux numériques par un module de conversion analogue/digitale 71. La conversion analogue/digitale 71 est suivie d'une étape de traitement mathématique par un module 72 au cours de laquelle les données numériques sont traitées selon la méthode décrite plus haut. A l'étape de traitement mathématique par le module 72 succède une étape de détermination de valeur numérique par un module 73 au cours de laquelle le résultat du traitement mathématique par le module 72 sera utilisé pour déterminer, à l'aide de la droite d'acquisition de position DAP illustrée à la figure 4, une valeur numérique correspondant à la position angulaire du rotor 10.

## ELECTRONIQUE EMBARQUEE

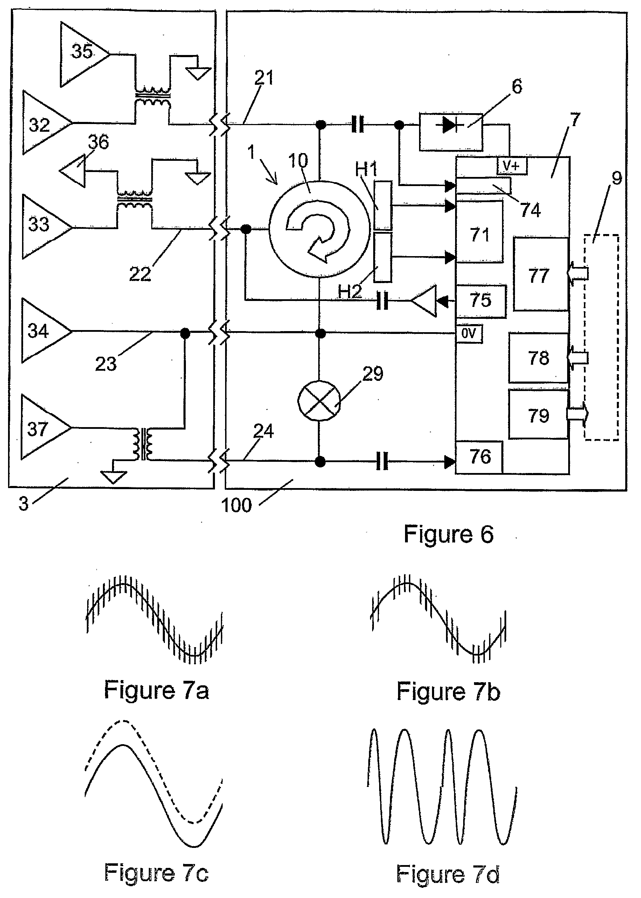

La figure 6 donne le schéma synoptique de l'électronique selon une version préférentielle de l'invention. Une partie de l'électronique représentée schématiquement est située sur le module électronique d'asservissement 3 servant en particulier à l'alimentation et la commande de l'instrument 100 tandis qu'une autre partie de l'électronique est embarquée dans l'instrument 100. La répartition des éléments électroniques entre le module électronique d'asservissement 3 et l'instrument 100 peut cependant être modifiée dans le cadre de l'invention.

Les éléments désignés par certains numéros de référence dans la figure 6 sont décrits ci-dessous:

32: Driver de puissance analogique de la phase I du moteur 1.

33: Driver de puissance analogique de la phase II du moteur 1.

34: Driver de puissance analogique de la phase III du moteur 1.

35: Amplificateur d'insertion sur la phase I de la haute fréquence, par exemple 2MHz, fournissant l'alimentation et le synchronisme du microcontrôleur 7 embarqué.

36: Amplificateur de réception des informations émises par le microcontrôleur 7 embarqué dans l'instrument.

37: Driver de puissance pour la lampe 29 et modulateur FSK pour l'émission de datas vers le microcontrôleur 7 embarqué.

7: Microcontrôleur embarqué comprenant de préférence les fonctions et/ou éléments suivants:

■ Synchro 74: pour avoir des transmissions d'informations non perturbées, il est important que celles-ci soient synchronisées avec la haute fréquence (par exemple à 2MHz) d'alimentation du microcontrôleur 7 embarqué.

■ Données output 75: selon une version préférentielle de l'invention, les informations émises par le microcontrôleur 7 embarqué travaillent à la moitié de la haute fréquence selon un principe décrit plus bas. Les informations émises par le microcontrôleur 7 embarqué travaillent ainsi par exemple à 1Mbauds, soit la moitié de 2MHz.

■ Données input 76: la lampe 29 est alimentée par une tension AC de 350kHz. Le transfert des données depuis le module électronique 3 vers le microcontrôleur 7 s'opère par modulation de fréquence selon la technique FSK, par exemple à une fréquence de 350kHz $\pm$ 25kHz.

■ Entrées/sorties auxiliaires 78, resp. 79: le microcontrôleur 7 est à même, en plus de la droite DAP, de transmettre des informations auxiliaires (analogiques ou digitales) provenant d'éléments de commande ou de mesure également embarqués.

29: Lampe.

9: Fonctions auxiliaires optionnelles, par exemple éléments de commande et de mesure, ou éléments actifs enclenchés par le module électronique d'asservissement 3, etc.

21: Conducteur de la phase I du moteur 1 sur lequel sont transmises la composante d'entraînement du moteur (signal basse fréquence) et la composante haute fréquence d'alimentation du microcontrôleur 7 embarquée (figure 7a).

22: Conducteur de la phase II du moteur 1 sur lequel sont transmises la composante d'entraînement du moteur et la composante par exemple à 1Mbauds des données transmises depuis le microcontrôleur 7 vers le module 3 (figure 7b).

23: Conducteur de la phase III du moteur 1. Cette phase est le conducteur neutre du point de vue haute fréquence. Le "0V" (gnd) du microcontrôleur 7 suit la composante d'entraînement du moteur représentée par la courbe inférieure de la figure 7c. La composante supérieure est l'exacte réplique de la composante inférieure additionnée de 5V et connectée à "V+" du microcontrôleur 7 de l'instrument 100. "V+" est généré par la haute fréquence redressée de la phase I, débarrassée de sa composante basse fréquence.

24: Conducteur d'alimentation de la lampe 29 et d'acheminement des informations transmises depuis le module 3 vers le microcontrôleur 7. La lampe 29 est alimentée en tension AC, régulée en courant, d'une fréquence d'environ 350kHz. Les informations à l'attention du microcontrôleur 7 embarqué dans l'instrument 100 sont codées par modulation de fréquence ($\pm$25kHz).

6: Redresseur permettant par exemple l'alimentation électrique du circuit électronique numérique embarqué dans l'instrument 100. Le redresseur 6 redresse par exemple un signal haute fréquence injecté par le module 3 sur l'un des conducteurs électriques, par exemple sur le conducteur 21. Le circuit électronique numérique embarqué, en particulier le microcontrôleur 7 est alors alimenté électriquement par la sortie du filtre de redressage 6.

## LES INFORMATIONS DU MICROCONTRÔLEUR AU MODULE

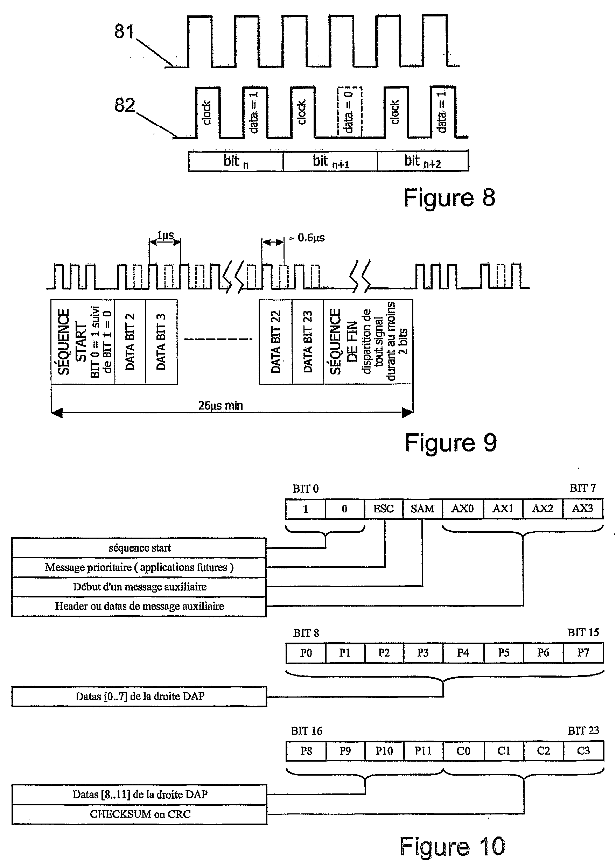

Entre le microcontrôleur 7 et le module électronique d'asservissement 3, la transmission est de type série. Les données sont donc transmises en série selon le principe illustré à la figure 8. Le signal 81 est la haute fréquence d'alimentation, par exemple à 2Mhz, transmise sur la phase I du moteur. Le signal 82 représente le flux d'informations, par exemple à 1 MBauds, transmis sur la phase II.

Il ressort de la figure 8 que quelle que soit la valeur du bit, elle est toujours précédée de l'impulsion clock. L'exiguïté de l'électronique embarquée ne permet pas de "clocker" le microcontrôleur par un quartz, mais par un circuit RC d'une stabilité relative. La technique ci-dessus maintient un parfait synchronisme en réception.

### Protocole de niveau 1 (niveau hardware)

En référence à la figure 9, un mot se compose par exemple de 24 bits, dont deux bits BIT0, BIT1 de la séquence de démarrage SEQUENCE START et 22 bits de données DATA BIT 2, ..., DATA BIT 23. La fin du mot est par exemple signalée par l'absence de signal durant au moins 2 bits, ce qui représente par exemple un intervalle SEQUENCE DE FIN de 2 $\mu$s.

D'autres longueurs de mot, séquences de démarrage et/ou de fin sont cependant parfaitement envisageable dans le cadre de l'invention.

### Protocole de niveau 2 (attribution des bits)

L'attribution des bits des messages envoyés par le microcontrôleur 7 embarqué vers le module électronique 3 est de préférence définie telle qu'illustré à la figure 10. D'autres modes d'attributions sont cependant parfaitement envisageables dans le cadre de l'invention. Le nombre de bits attribués à chaque fonction et/ou le nombre et le type de fonctions peuvent en particulier être modifiés selon les besoins.

#### Les messages auxiliaires

On entend par messages auxiliaires, toute information ne concernant pas la position angulaire du rotor. Ces informations sont générées dans l'instrument par les fonctions auxiliaires 9 de la figure 7.

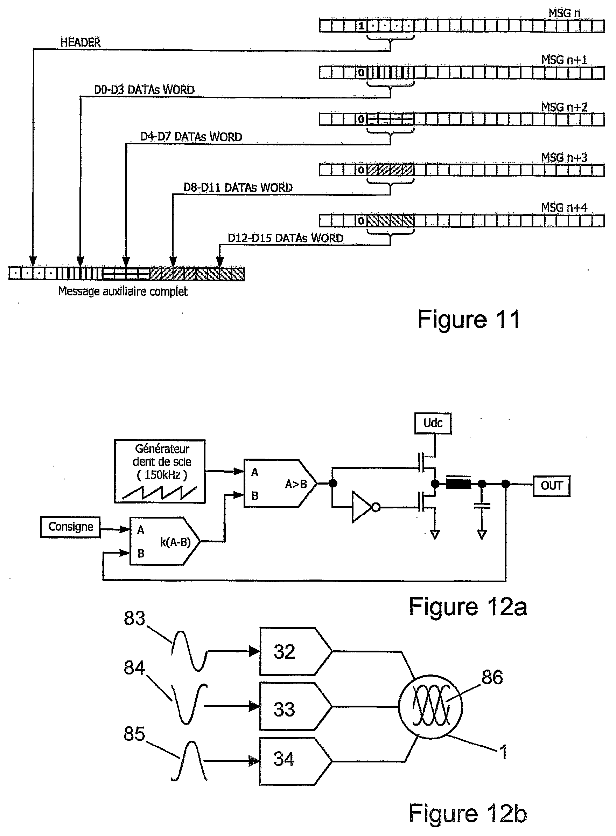

Chaque message auxiliaire est par exemple composé d'un HEADER, par exemple de 4bits, et d'un DATAs WORD, par exemple de 16bits, transmis selon une version préférentielle comme le montre la figure 11. D'autres formes de transmission et/ou de codage des messages auxiliaires sont cependant envisageables dans le cadre de l'invention.

## LES INFORMATIONS DU MODULE VERS LE MICROCONTRÔLEUR

Selon une version préférentielle de l'invention, la communication du module électronique d'asservissement 3 vers le microcontrôleur 7 est effectuée par modulation de fréquence sur le conducteur lampe 24.

La transmission d'informations du module électronique d'asservissement 3 vers le microcontrôleur 7 peut cependant également être effectué sur un autre conducteur 21, 22, 23 et à l'aide d'autres méthodes de modulation.

## L'ASSERVISSEMENT DU MOTEUR

### L'entraînement du moteur de l'instrument

Selon la version préférentielle de l'invention, le moteur 1 est entraîné par trois drivers analogiques 32, 33, 34 fournissant des signaux sinusoïdaux 83, 84, 85 formant un champ tournant 86 déphasé de 120° d'amplitude et de fréquence variables (figure 12b). Le choix d'un tel entraînement procure deux avantages importants:

- Meilleur rendement du moteur 1 sur toute la gamme de vitesse.

- Rotation très "douce", essentiellement aux basses vitesses.

Dans une variante, le système fonctionne avec des drivers "tout ou rien".

Le schéma de principe des drivers selon une variante de l'invention est illustré à la figure 12a. D'autres types de drivers sont cependant envisageables dans le cadre de l'invention.

### L'asservissement de position

#### Principe de base de la mise en rotation

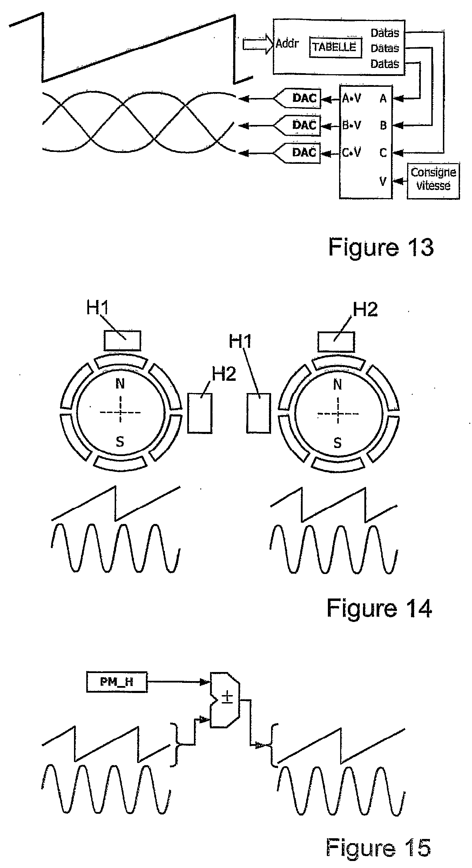

Dans son principe de base, la mise en rotation du moteur selon une version préférentielle de l'invention est simple. Il suffit de créer une tabelle qui, à partir de l'adressage donné par la valeur numérique de la droite DAP, fournit trois valeurs de sinus déphasées de 120°. La vitesse du moteur est alors définie par l'amplitude commune aux trois sinus comme le schématise la figure 13.

#### Compensation de la position géométrique des capteurs de Hall

La relation entre la phase des signaux de commande analogique et la phase de la droite DAP dépend de la position des capteurs de Hall H1, H2, comme illustré à la figure 14 qui montre deux dispositions possibles. Pour corriger cet effet, il faut de préférence additionner un paramètre signé ($\pm$ PM_H) sur la droite DAP en tenant compte du modulo[360°]. La figure 15 illustre le circuit de compensation selon une variante de l'invention.

#### Compensation de l'angle de réactualisation $\alpha_R$

L'acquisition de la position angulaire $\alpha$ du rotor 10 et les calculs qui en découlent prennent un certains temps, typiquement environ $50\mu s$, durant lequel le moteur tourne. Cela signifie que la réactualisation des drivers 32, 33, 34 du moteur 1 dépend d'une lecture de position angulaire $\alpha$ qui est dépassée. Cette différence de position ou angle de réactualisation $\alpha_R$, est d'autant plus grand que la vitesse du moteur 1 est grande; en fait, elle est directement proportionnelle à la vitesse du moteur.

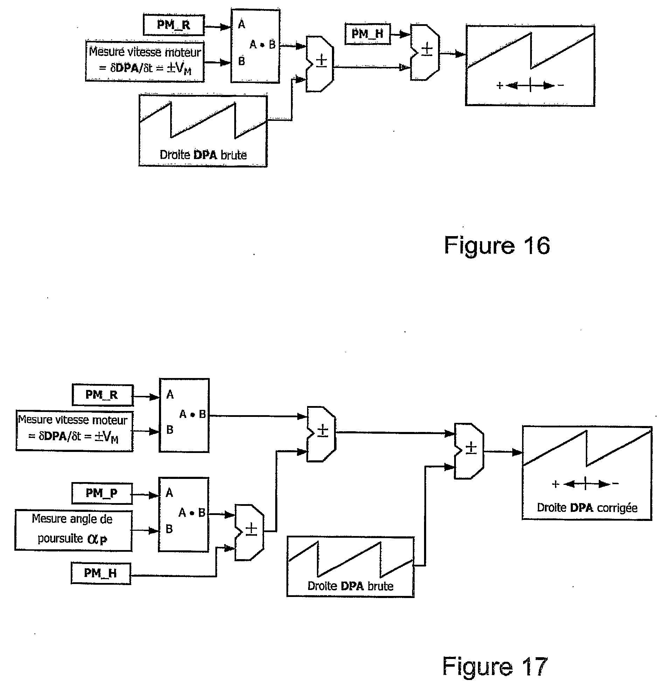

Une solution préférentielle pour compenser cette différence consiste à additionner à la droite DAP modulo[360°] une valeur proportionnelle à la vitesse signée ($\pm$ V$_M$). La constante de proportionnalité est définie par le paramètre PM_R. La figure 16 montre le dispositif de compensation selon une variante de l'invention.

#### Compensation de l'angle de poursuite $\alpha_P$

L'angle de poursuite $\alpha_P$ est l'angle formé par le vecteur magnétique du champ tournant 86 et celui du rotor 10. Cet angle augmente proportionnellement à la vitesse et au couple. On le calcule en effectuant :

$$\alpha_P = \text{Position de consigne - position DAP}$$

La constante de proportionnalité est définie par le paramètre PM_P et la figure 17 montre l'ensemble des circuits de compensation selon une variante préférentielle de l'invention. Il est cependant envisageable, dans le cadre de l'invention, de n'implémenter que certains des circuits de compensation décrits ci-dessus, de les implémenter à l'aide de circuits différents, ou par logiciel. D'autres circuits de compensation non décrits ici sont en outre envisageables dans le cadre de l'invention.

L'invention est décrite ci-dessus dans le cadre d'un dispositif dentaire comprenant quatre conducteurs électriques entre le module d'asservissement 3 et l'instrument 100. L'invention s'applique cependant également à d'autres dispositifs chirurgicaux comprenant par exemple un nombre différents de contacts électriques entre le module d'asservissement 3 et l'instrument 100 sur lesquelles peuvent être transmises des données et/ou l'alimentation de l'électronique embarquée.

Ainsi qu'expliqué au moins en partie plus haut et en référence aux figures 1 à 19, le dispositif chirurgical, notamment dentaire, de l'invention comporte ainsi, dans certaines variantes de l'invention, une ou plusieurs des caractéristiques récitées ci-dessous.

L'instrument 100 comprend un circuit électronique numérique 4 embarqué comportant au moins un convertisseur analogique-numérique, par exemple sous la forme d'un module 71, pour convertir les signaux de sortie analogiques du ou des capteurs H1, H2 en signaux numériques traités et transmis au module électronique d'asservissement 3. Le circuit électronique numérique 4 comporte par exemple un processeur 7 comprenant le module de conversion analogique-numérique 71. Le processeur 7 permet plus généralement de traiter et transmettre au module électronique d'asservissement 3 des données obtenues à partir des signaux de sortie cH1, sH2 sous le contrôle d'un programme. Le circuit électronique 4 calcule par exemple la tangente des signaux en cosinus cH1 et en sinus sH2 fournis par les deux capteurs H1, H2. Le circuit numérique calcule de préférence la tangente ramenée dans une zone de 0 à 45°, un ou plusieurs bits indiquant par exemple la zone utilisée.

Le circuit électronique 4 calcule et transmet ainsi par exemple au module électronique d'asservissement 3 la position angulaire $\alpha$ instantanée du rotor 10 du moteur 1. Le circuit électronique 4 est par exemple agencé pour transmettre périodiquement, à intervalles réguliers, au module 3 des données numériques dépendant des signaux de sortie cH1, sH2, les données numériques étant de préférence modulées numériquement sur un ou plusieurs des fils électriques 21, 22, 23, 24 du tuyau 2. Les données numériques sont par exemple transmises en série sous forme de mots sur exactement un des fils électriques, par exemple sur un des conducteurs de phase alimentant le moteur électrique 1, la fréquence utilisée pour transmettre les mots étant supérieure à la fréquence d'alimentation du moteur 1. Selon une variante, les mots numériques incluent des indications auxiliaires fournies par des capteurs ou des interrupteurs 9 autres que les capteurs de position angulaire H1, H2. Les indications auxiliaires incluent par exemple une ou plusieurs des indications suivantes:

- des indications fournies par un capteur de température dans l'instrument 100,

- des indications relatives au type d'outil connecté à l'instrument, le type d'outil étant par exemple déterminé automatiquement,

- des indications relatives à la durée d'utilisation d'une ampoule 29 dans l'instrument,

- des indications relatives à l'actionnement d'une gâchette, d'un interrupteur ou d'un dispositif de réglage sur ledit instrument,

- des indications relatives à la circulation de liquide et/ou d'air par le raccord flexible 2,

- etc.

L'instrument 100 comporte par exemple un interrupteur à deux ou plusieurs positions fournissant un signal de commande numérique interprété par le circuit électronique numérique 4.

Le circuit électronique numérique 4 fournit de préférence plusieurs fois par tour du rotor 10 au module 3 des données numériques indiquant la position angulaire $\alpha$ absolue du rotor 10.

Le module électronique d'asservissement 3 est de préférence agencé pour transmettre au circuit électronique numérique 4 des données numériques de commande modulées numériquement sur les fils électriques 21, 22, 23, 24 du raccord flexible 2. Les données numériques de commande sont par exemple transmises en série sous forme de mots sur exactement un des fils électriques.

Le module électronique d'asservissement 3 comporte de préférence trois drivers de puissance 32, 33, 34 pour alimenter les trois phases du moteur 1. Les trois drivers de puissance 32, 33, 34 sont par exemple des drivers analogiques fournissant trois signaux analogiques déphasés de 120° et de fréquence variable.

De préférence, le module électronique d'asservissement 3 contrôle la vitesse de rotation du moteur 1. Il contrôle par exemple le couple du moteur 1. De préférence, le module électronique 3 asservit cependant à la fois la vitesse de rotation et le couple du moteur 1, dans quatre quadrants.

Le module d'asservissement 3 effectue en outre de préférence une correction de la valeur de consigne pour tenir compte des erreurs de positionnement des capteurs de Hall H1, H2 déterminées lors du calibrage du dispositif. Il effectue de préférence également une correction de la valeur de consigne pour tenir compte de la rotation du rotor 10 au cours de la mesure et du calcul et/ou une correction de la valeur de consigne pour compenser l'angle de poursuite entre le champ tournant statorique et le champ du rotor 10. La correction apportée pour compenser l'angle de poursuite est de préférence proportionnelle à la tension appliquée sur les bobines.

Le module d'asservissement 3 comporte par exemple un processeur contrôlé par un programme pour contrôler le fonctionnement de l'instrument 100. Le programme permet par exemple de définir des cycles d'usinage avec l'instrument 100, les cycles d'usinage comportant par exemple plusieurs allers et retours avec l'instrument 100 ou l'outil pour briser des copeaux. Selon une variante, le programme permet de contrôler la profondeur d'usinage avec l'outil.

L'instrument 100 comporte par exemple un capteur CCD fournissant des données traitées par le circuit numérique 4 et transmises au module électronique d'asservissement 3.

## Revendications

1. Dispositif chirurgical, notamment dentaire, comportant:

   un instrument (100) muni d'un moteur rotatif (1) pour l'entraînement d'un outil et d'au moins un capteur (H1, H2) pour déterminer au moins un paramètre de fonctionnement dudit moteur (1), et d'un circuit électronique numérique (4) pour traiter les signaux de sortie (cH1, sH2) dudit au moins un capteur (H1, H2),
   un module électronique (3) d'asservissement dudit moteur (1),
   un raccord flexible (2) pour relier électriquement ledit instrument (100) avec ledit module électronique (3), ledit raccord flexible comportant des fils électriques (21, 22, 23, 24) pour alimenter électriquement ledit moteur (1) et/ou un accessoire électrique dudit instrument (100) ou de la pièce à main dudit instrument (100),
   **caractérisé en ce que** ledit circuit électronique numérique (4) est agencé pour transmettre périodiquement, à intervalles réguliers audit module électronique (3) au travers dudit raccord flexible (2) des données numériques dépendant desdits signaux de sortie (cH1, sH2), lesdites données numériques étant modulées numériquement sur au moins un desdits fils électriques (21, 22, 23, 24) dudit raccord flexible (2).

2. Le dispositif de la revendication 1, dans lequel ledit moteur (1) est un moteur sans collecteur, par exemple un moteur triphasé comportant trois enroulements statoriques (L1, L2, L3) montés en étoile.

3. Le dispositif de l'une des revendications 1 à 2, dans lequel ledit moteur rotatif (1) comporte un rotor (10), et dans lequel ledit au moins un capteur (H1, H2) permet de déterminer la position angulaire ($\alpha$) instantanée dudit rotor (10).

4. Le dispositif de la revendication 3, dans lequel deux capteurs de Hall ou magnétorésistifs (H1, H2) déphasés de 90° fournissent deux signaux (cH1, sH2) en quadrature dont la phase dépend de la position angulaire ($\alpha$) dudit rotor (10).

5. Le dispositif de l'une des revendications 1 à 4, dans lequel ledit raccord flexible (2) comporte exactement quatre fils électriques (21, 22, 23, 24) pour relier électriquement ledit module électronique d'asservissement (3) audit instrument (100),
   trois desdits quatre fils électriques (21, 22, 23, 24) sont utilisés pour alimenter électriquement trois enroulements statoriques (L1, L2, L3, L4) dudit moteur (1),
   le quatrième fil électrique étant relié électriquement à un accessoire électrique dudit instrument (100) ou de la pièce à main dudit instrument (100), par exemple à une lampe (29),
   ledit raccord flexible (2) comportant en outre des tuyaux (25, 26, 27, 28) pour le passage d'air et/ou de liquide.

6. Le dispositif de l'une des revendications 1 à 5, dans lequel ledit circuit électronique numérique (4) comporte au moins un convertisseur analogique-numérique (71) pour convertir les signaux de sortie analogiques dudit au moins un capteur (H1, H2) en signaux numériques traités et transmis audit module électronique d'asservissement (3).

7. Le dispositif de l'une des revendications 1 à 6, dans lequel ledit circuit électronique numérique (4) comporte un processeur (7) pour traiter et transmettre audit module électronique d'asservissement (3) des données obtenues à partir desdits signaux de sortie (cH1, sH2) sous le contrôle d'un programme.

8. Le dispositif de l'une des revendications 1 à 7, dans lequel ledit circuit électronique (4) calcule la tangente de deux signaux (cH1, sH2) en quadrature fournis par deux dits capteurs (H1, H2).

9. Le dispositif de la revendication 8, dans lequel ledit circuit numérique (4) calcule ladite tangente ramenée dans une zone de 0 à 45°, un ou plusieurs bits indiquant la zone utilisée.

10. Le dispositif de l'une des revendications 1 à 9, dans lequel ledit circuit électronique (4) calcule et transmet audit module électronique d'asservissement (3) la position angulaire ($\alpha$) instantanée du rotor (10) dudit moteur (1).

11. Le dispositif de l'une des revendications 1 à 10, dans lequel lesdites données numériques sont transmises en série sous forme de mots sur exactement un desdits fils électriques (21, 22, 23, 24), par exemple sur un des conducteurs de phase (21, 22, 23) alimentant ledit moteur électrique (1), la fréquence utilisée pour transmettre lesdits mots étant supérieure à la fréquence d'alimentation dudit moteur (1).

12. Le dispositif de la revendication 11, dans lequel lesdits mots numériques incluent des indications auxiliaires fournies par des capteurs ou des interrupteurs (9) autres que les capteurs de position angulaire, par exemple :

des indications fournies par un capteur de température dans ledit instrument, et/ou
des indications relatives au type d'outil connecté à ledit instrument (100), ledit type d'outil étant déterminé automatiquement, et/ou
des indications relatives à la durée d'utilisation d'une ampoule (29) dans ledit instrument (100), et/ou
des indications relatives à l'actionnement d'une gâchette, d'un interrupteur ou d'un dispositif de réglage sur ledit instrument (100), et/ou
des indications relatives à la circulation de liquide et/ou d'air par ledit raccord flexible (2).

13. Le dispositif de l'une des revendications 1 à 12, dans lequel ledit instrument (100) comporte un interrupteur à deux plusieurs positions fournissant un signal de commande numérique interprété par ledit circuit électronique numérique (4).

14. Le dispositif de l'une des revendications 1 à 13, dans lequel ledit circuit électronique numérique (4) fournit plusieurs fois par tour du rotor (10) audit module (3) des données numériques indiquant la position angulaire ($\alpha$) absolue dudit rotor (10).

15. Le dispositif de l'une des revendications 1 à 14, dans lequel ledit instrument comporte un redresseur (6) pour redresser un signal haute fréquence injecté par ledit module (3) sur l'un des fils électriques (21, 22, 23, 24) dudit raccord (2), ledit circuit électronique numérique (4) étant alimenté électriquement par la sortie dudit filtre de redressage (6).

16. Le dispositif de l'une des revendications 1 à 15, dans lequel ledit module électronique d'asservissement (3) est agencé pour transmettre audit circuit électronique numérique (4) des données numériques de commande modulées numériquement sur des fils électriques (21, 22, 23, 24) dudit raccord flexible (2), lesdites données numériques de commande étant transmises en série sous forme de mots sur exactement un desdits fils électriques (21, 22, 23, 24).

17. Le dispositif de l'une des revendications 1 à 16, ledit instrument (100) comportant un accessoire électrique, par exemple une lampe (29), dont l'une des bornes est reliée :

à un des fils (21, 22, 23, 24) dudit raccord flexible (2) dédié à cet usage, et/ou
à un des conducteurs de phase (21, 22, 23) dudit moteur (1), et/ou
au point neutre entre les phases dudit moteur (1), et/ou
au dit circuit électronique numérique (4) commandant son fonctionnement.

18. Le dispositif de l'une des revendications 1 à 17, dans lequel ledit module électronique d'asservissement (3) comporte trois drivers de puissance (32, 33, 34) pour alimenter les trois phases dudit moteur (1).

19. Le dispositif de la revendication 18, dans lequel lesdits trois drivers de puissance (32, 33, 34) sont des drivers analogiques fournissant trois signaux analogiques (83, 84, 85) déphasés de 120° et de fréquence variable.

20. Le dispositif de l'une des revendications 1 à 19, dans lequel ledit module électronique d'asservissement (3) contrôle la vitesse de rotation dudit moteur (1) et/ou le couple dudit moteur (1).

21. Le dispositif de l'une des revendications 1 à 20, dans lequel ledit module d'asservissement (3) effectue une correction de la valeur de consigne pour tenir compte des erreurs de positionnement des capteurs de Hall (H1, H2) déterminées lors du calibrage du dispositif.

22. Le dispositif de l'une des revendications 1 à 21, dans lequel ledit module d'asservissement (3) effectue une correction de la valeur de consigne pour tenir compte de la rotation du rotor au cours de la mesure et du calcul et/ou pour compenser l'angle de poursuite entre le champ tournant statorique et le champ du rotor (10).

23. Le dispositif de la revendication 22, dans lequel la correction apportée pour compenser ledit angle de poursuite est proportionnelle à la tension appliquée sur lesdites bobines.

24. Le dispositif de l'une des revendications 1 à 23, dans lequel ledit module d'asservissement (3) comporte un processeur contrôlé par un programme pour contrôler le fonctionnement dudit instrument (100), ledit programme permettant de définir des cycles d'usinage avec ledit outil (100), par exemple des cycles d'usinage comportant plusieurs allers et retours avec ledit outil (100) pour briser les copeaux.

25. Le dispositif de l'une des revendications 1 à 24, dans lequel un blindage est prévu entre les enroulements statoriques dudit moteur (1) et ledit circuit électronique numérique (4).

26. Le dispositif de l'une des revendications 1 à 25, dans lequel ledit au moins un capteur (H1, H2) est logé dans un évidement dans les tôles statoriques dudit moteur (1).

27. Le dispositif de l'une des revendications 1 à 26, dans lequel ledit au moins un capteur (H1, H2) est logé dans un évidement entre le rotor (10) et le stator.

28. Le dispositif de l'une des revendications 1 à 27, dans lequel ledit circuit électronique numérique (4) est monté sur une carte de circuit imprimée perpendiculaire à l'axe de rotation dudit moteur (10), au moins un capteur (H1, H2) étant de préférence monté sur ladite carte de circuit imprimé.

29. Le dispositif de l'une des revendications 1 à 28, dans lequel ledit instrument (100) comporte un capteur CCD fournissant des données traitées par ledit circuit numérique (4) et transmises audit module électronique d'asservissement (3).

**Patentansprüche**

1. Chirurgische Vorrichtung, insbesondere Dentalvorrichtung, mit:

einem Instrument (100) mit einem Drehmotor (1) zum Antrieb eines Werkzeugs und mit mindestens einem Sensor (H1, H2) zur Bestimmung zumindest eines Betriebsparameters des besagten Motors (1), und mit einer digitalen elektronischen Schaltung (4) zur Verarbeitung der Ausgangssignale (cH1, sH2) des besagten mindestens einen Sensors (H1, H2),
einem elektronischen Servomodul (3) des besagten Motors (1),
einem flexiblen Verbindungsstück (2) zur elektrischen Verbindung des besagten Instruments (100) mit dem besagten elektronischen Modul (3), wobei das besagte flexible Verbindungsstück elektrische Leitungen (21, 22, 23, 24) zur elektrischen Speisung des besagten Motors (1) und/oder ein elektrisches Zubehörteil des besagten Instruments (100) oder des Handstücks des besagten Instruments (100) aufweist,
**dadurch gekennzeichnet, dass** die besagte digitale elektronische Schaltung (4) derart angeordnet ist, um periodisch, in regelmässigen Abständen, über das besagte flexible Verbindungsstück (2) an das besagte elektronische Modul (3) digitale Daten in Abhängigkeit der besagten Ausgangssignale (CH1, SH2) zu übertragen, wobei die besagten digitale Daten auf mindestens einer der besagten elektrischen Leitungen (21, 22, 23, 24) des besagten flexiblen Verbindungsstücks (2) moduliert werden.

**2.** Vorrichtung gemäss Anspruch 1, worin der Motor (1) ein bürstenloser Motor ist, beispielsweise ein Dreiphasenmotor mit drei sternförmig angeordneten Statorwicklungen (L1, L2, L3).

**3.** Vorrichtung gemäss einem der Ansprüche 1 bis 2, worin der besagte Drehmotor (1) einen Rotor (10) umfasst, und worin der besagte mindestens eine Sensor (H1, H2) es erlaubt, die momentane Winkelposition ($\alpha$) des besagten Rotors (10) zu bestimmen.

**4.** Vorrichtung gemäss Anspruch 3, worin zwei um 90 Grad phasenverschobene Hallsensoren oder magnetoresistive Sensoren (H1, H2) zwei Quadratursignale (cH1, sH2) liefern, deren Phase von der Winkelstellung ($\alpha$) des besagten Rotors (10) abhängig ist.

**5.** Vorrichtung gemäss einem der Ansprüche 1 bis 4, worin das besagte flexible Verbindungsstück (2) genau vier elektrische Leitungen (21, 22, 23, 24) aufweist zur elektrischen Verbindung (3) des besagten elektronischen Servomoduls (3) mit dem besagten Instrument (100),
wobei drei der besagten vier elektrischen Leitungen (21, 22, 23, 24) benutzt werden, um die drei Statorwicklungen (L1, L2, L3, L4) des besagten Motors (1) mit elektrischer Energie zu versorgen,
wobei die vierte elektrische Leitung elektrisch mit einem elektrischen Zubehörteil des besagten Instruments (100) oder einem Handstück des besagten Instruments (100), beispielsweise mit einer Lampe (29), verbunden ist,
wobei das flexible Verbindungsstück (2) zudem Schläuche (25, 26, 27, 28) für den Durchgang von Luft und/oder Flüssigkeit aufweist.

**6.** Vorrichtung gemäss einem der Ansprüche 1 bis 5, worin die besagte digitale elektronische Schaltung (4) mindestens einen Analog-Digital-Wandler (71) umfasst zur Umwandlung der analogen Ausgangssignale des besagten mindestens einen Sensors (H1, H2) in verarbeitete und an das besagte elektronische Steuermodul übermittelte (3) digitalen Signale.

**7.** Vorrichtung gemäss einem der Ansprüche 1 bis 6, worin die besagte digitale elektronische Schaltung (4) mindestens einen Prozessor (7) umfasst zur Verarbeitung und Übertragung an das besagte elektronische Servomodul (3) von Daten, welche auf der Basis der besagten Ausgangssignalen (cH1, sH2) unter der Steuerung eines Programms erhalten wurden.

**8.** Vorrichtung gemäss einem der Ansprüche 1 bis 7, worin die elektronische Schaltung (4) die Tangente die beiden durch die besagten zwei Sensoren (H1, H2) gelieferten Quadratursignale (cH1, sH2) berechnet.

**9.** Vorrichtung gemäss Anspruch 8, worin die besagte elektronische Schaltung (4) die reduzierte Tangente in einem Bereich von 0 bis 45 Grad berechnet, wobei ein oder mehrere Bits den verwendeten Bereich anzeigen.

**10.** Vorrichtung gemäss einem der Ansprüche 1 bis 9, worin die besagte elektronische Schaltung (4) die momentane Winkelstellung ($\alpha$) des Rotors (10) des besagten Motors (1) berechnet und an das elektronische Steuermodul (3) übermittelt.

**11.** Vorrichtung gemäss einem der Ansprüche 1 bis 10, worin die besagten digitalen Daten seriell in Form von Worten auf genau einen der besagten elektrischen Leitungen (21, 22, 23, 24) übertragen werden, beispielsweise auf einem der den besagten Elektromotor (1) speisenden Phasenleiter (21, 22,23), wobei die zur Übertragung der besagten Worte verwendete Frequenz grösser als die Versorgungsfrequenz des Motors (1) ist.

**12.** Vorrichtung gemäss Anspruch 11, worin die besagten digitalen Wörter Hilfsangaben umfassen, die von sich von den Winkelpositionssensoren unterscheidenden Sensoren oder Schaltern (9) geliefert werden, beispielsweise von einem Temperatursensor im besagten Instrument gelieferte Angaben, und/oder
Angaben bezüglich der Art des mit dem besagten Werkzeug (100) verbundenen Instruments, wobei der Werkzeugtyp automatisch ermittelt wird, und/oder
Angaben in Bezug auf die Dauer der Verwendung einer Glühlampe (29) im besagten Instrument (100), und/oder
Angaben über die Betätigung eines Auslösers, eines Schalters oder einer Einstellvorrichtung auf das besagte Instrument (100), und/oder
Angaben betreffend die Zirkulation von Flüssigkeit und/oder Luft durch das besagte flexible Verbindungsstück.

**13.** Vorrichtung gemäss einem der Ansprüche 1 bis 12, worin das besagte Instrument (100) einen Schalter mit zwei oder mehreren Stellungen umfasst, welcher ein digitales Steuersignal liefert, das von der besagten elektronischen Schaltung (4) interpretiert wird.

14. Vorrichtung gemäss einem der Ansprüche 1 bis 13, worin die besagte digitale elektronische Schaltung (4) mehrere Male pro Umdrehung des Rotors (10) dem Modul (3) digitale Daten liefert, welche die absolute Winkelposition ($\alpha$) des Rotors (10) anzeigen.

15. Vorrichtung gemäss einem der Ansprüche 1 bis 14, worin das besagte Instrument einen Gleichrichter (6) umfasst, um ein durch das besagte Modul (3) injiziertes Hochfrequenzsignal auf einer der elektrischen Leitungen (21, 22, 23, 24) des besagten Verbindungsstücks (2) gleichzurichten, wobei die besagte digitale elektronische Schaltung (4) elektrisch vom Ausgang des Richterfilters (6) gespeist wird.

16. Vorrichtung gemäss einem der Ansprüche 1 bis 15, worin das besagte elektronische Servomodul (3) derart angeordnet ist, um an die besagte elektronische Schaltung (4) digital auf den elektrischen Leitungen (21, 22, 23, 24) des besagten Verbindungsstücks (2) modulierte digitale Steuerungsdaten zu übertragen,
wobei die besagten digitalen Steuerungsdaten seriell in Form von Worten auf genau einer der besagten elektrischen Leitungen (21, 22, 23, 24) übertragen werden.

17. Vorrichtung gemäss einem der Ansprüche 1 bis 16, worin das besagte Instrument (100) ein elektrisches Zubehörteil aufweist, beispielsweise eine Lampe (29), wovon einer der Anschlüsse verbunden ist:

    mit der dazu vorgesehenen einen der Leitungen (21, 22, 23, 24) des besagten flexiblen Verbindungsstücks (2), und/oder
    mit einem Phasenleiter (21, 22, 23) des besagten Motors (1), und/oder
    mit einem neutralen Punkt zwischen den Phasen des besagten Motors (1), und/oder
    mit der besagten digitalen elektronischen Schaltung (4), welche ihren Betrieb steuert.

18. Vorrichtung gemäss einem der Ansprüche 1 bis 17, worin das besagte elektronische Servomodul (3) drei Leistungsstufen (32, 33, 34) zur Versorgung der drei Phasen des besagten Motors (1) aufweist.

19. Vorrichtung gemäss Anspruch 18, worin die drei besagten Leistungsstufen (32, 33, 34) analoge Treiber sind, welche drei um 120 Grad phasenverschobene Analogsignale (83, 84, 85) mit variabler Frequenz liefern.

20. Vorrichtung nach einem der Ansprüche 1 bis 19, worin das besagte Steuerelektronikmodul (3) die Geschwindigkeit der Drehung des besagten Motors (1) und/oder das Drehmoment des besagten Motors (1) steuert.

21. Vorrichtung gemäss einem der Ansprüchen 1 bis 20, worin das besagte elektronische Servomodul (3) eine Korrektur des Sollwertes durchführt, um den während der Kalibrierung des Gerätes bestimmten Positionierungsfehlern der Hallsensoren (H 1, H2) Rechnung zu tragen.

22. Vorrichtung gemäss einem der Ansprüchen 1 bis 21, worin das besagte elektronische Servomodul (3) eine Korrektur des Sollwertes durchführt, um der Drehung des Motors während der Messung und der Berechnung Rechnung zu tragen, und/oder um die Winkelnachführung zwischen dem Statordrehfeld und dem Rotorfeld (10) zu kompensieren.

23. Vorrichtung gemäss Anspruch 22, worin die Korrektur zur Kompensierung der Winkelnachführung proportional zur angelegten Spannung an die besagten Spulen ist.

24. Vorrichtung gemäss einem der Ansprüchen 1 bis 23, worin das besagte elektronische Servomodul (3) einen durch ein Programm zur Steuerung des Betriebs des besagten Instruments (100) kontrollierten Prozessor aufweist, wobei das besagte Programm es erlaubt, Bearbeitungszyklen mit dem besagten Werkzeug (100) zu definieren, beispielsweise Bearbeitungszyklen mit einigem Hin und Her mit dem besagten Werkzeug (100) für den Spanbruch.

25. Vorrichtung gemäss einem der Ansprüche 1 bis 24, worin eine Abschirmung zwischen den Statorwicklungen des besagten Motors (1) und der besagten digitalen elektronischen Schaltung (4) vorgesehen ist.

26. Vorrichtung gemäss einem der Ansprüche 1 bis 25, worin der besagte mindestens eine Sensor (H1, H2) in einer Aussparung in den Statorblechen des besagten Motors (1) untergebracht ist.

27. Vorrichtung gemäss einem der Ansprüche 1 bis 26, worin der besagte mindestens eine Sensor (H1, H2) in einer Aussparung zwischen dem Rotor (10) und dem Stator untergebracht ist.

28. Vorrichtung gemäss einem der Ansprüche 1 bis 27, worin die besagte digitale elektronische Schaltung (4) auf einer Leiterplatte senkrecht zur Drehachse des besagten Motors (10) montiert ist, wobei vorzugsweise mindestens ein Sensor (H1, H2) auf der besagten Leiterplatte angebracht ist.

29. Vorrichtung gemäss einem der Ansprüche 1 bis 28, worin das besagte Instrument (100) einen CCD-Sensor umfasst, welcher von der besagten digitalen Schaltung (4) verarbeitete Daten liefert und dem besagten elektronischen Servomodul (3) überträgt.

**Claims**

1. A surgical device, notably a dental device, comprising:

   an instrument (100) provided with a rotary motor (1) for driving a tool and at least one sensor (H1, H2) for determining at least one operating parameter of said motor (1), and a digital electronic circuit (4) for processing the output signals (cH1, sH2) of said at least one sensor (H1, H2),
   an electronic servo-control module (3) of said motor (1),
   a flexible connection (2) for electrically connecting said instrument (100) to said electronic module (3), said flexible connection comprising electric leads (21, 22, 23, 24) for electrically feeding said motor (1) and/or an electric accessory of said instrument (100) or of the hand part of said instrument (100),
   **characterized in that** said digital electronic circuit (4) is configured for sending, periodically, at regular intervals, to said electronic module (3) through said flexible connection (2) digital data depending on said output signals (cH1, sH2), said digital data being modulated digitally on the electric leads (21, 22, 23, 24) of said flexible connection (2).

2. The device of claim 1, wherein said motor (1) is a collectorless motor, for example a three-phased motor having three stator windings (L1, L2, L3) mounted in a star.

3. The device of one of claims 1 to 2, wherein said rotary motor (1) has a rotor (10) and wherein said at least one sensor (H1, H2) allows the instantaneous angular position ($\alpha$) of said rotor (10) to be determined.

4. The device of claim 3, wherein two Hall sensors or magnetoresistive sensors (H1, H2) phase-shifted by 90° supply two signals (cH1, sH2) in quadrature whose phase depends on the angular position ($\alpha$) of said rotor (10).

5. The device of one of claims 1 to 4, wherein said flexible connection (2) comprises exactly four electric leads (21, 22, 23, 24) for electrically connecting said electronic servo-control module (3) to said instrument (100),
   three of said four electric leads (21, 22, 23, 24) are used for electrically feeding three stator windings (L1, L2, L3, L4) of said motor (1),
   the fourth electric lead being connected electrically to an electric accessory of said instrument (100) or of the hand part of said instrument (100), for example to a lamp (29),
   said flexible connection (2) including among others tubes (25, 26, 27, 28) for the passage of air and/or liquid.

6. The device of one of claims 1 to 5, wherein said digital electronic circuit (4) comprises at least one analog-digital converter (71) for converting the analog output signals of said at least one sensor (H1, H2) into digital signals processed and transmitted to said electronic servo-control module (3).

7. The device of one of claims 1 to 6, wherein said digital electronic circuit (4) comprises a processor (7) for processing and transmitting to said electronic servo-control module (3) data obtained from said output signals (cH1, sH2) under control of a program.

8. The device of one of claims 1 to 7, wherein said electronic circuit (4) computes and transmits the tangent of two signals (cH 1, sH2) in quadrature delivered by said two sensors (H1, H2).

9. The device of claim 8, wherein said electronic circuit (4) computes said tangent conducted in a zone from 0 to 45°, one or more bits indicating the used zone.

10. The device of one of claims 1 to 9, wherein said electronic circuit (4) computes and transmits to said electronic servo-control module (3) the instantaneous angular position ($\alpha$) of said rotor (10) of said motor (1).

11. The device of one of claims 1 to 10, wherein said digital data are transmitted serially in the form of words on exactly one of said electric leads (21, 22, 23, 24), e.g. on one of the phase conductors (21, 22, 23) feeding said electric motor (1), the frequency used for transmitting said words being greater than the feed frequency of said motor (1).

12. The device of claim 11, wherein said digital words include auxiliary indications supplied by sensors or switches (9) other than the angular position sensors, for example:

   indications supplied by a temperature sensor in said instrument, and/or
   indications relative to the type of tool connected to said instrument (100), said type of tool being determined automatically, and/or
   indications relative to the duration of use of a bulb (29) in said instrument (100), and/or
   indications relative to the actuating of a trigger, switch or regulating device on said instrument (100), and/or
   indications relative to the circulation of liquid and/or air through said flexible connection (2).

13. The device of one of claims 1 to 12, wherein said instrument (100) includes a switch having two or more positions, supplying a digital command signal interpreted by said digital electronic circuit (4).

14. The device of one of claims 1 to 13, wherein said digital electronic circuit (4) supplies several times per turn of the rotor (10) to said module (3) digital data indicating the absolute angular position ($\alpha$) of said rotor (10).

15. The device of one of claims 1 to 14, wherein said instrument comprises a rectifier (6) for rectifying a high frequency signal injected by said module (3) on one of the electric leads (21, 22, 23, 24) of said connection (2), said digital electronic circuit (4) being electrically fed by the output of said rectifying filter (6).

16. The device of one of claims 1 to 15, wherein said electronic servo-control module (3) is arranged for transmitting to said digital electronic circuit (4) digital control data modulated digitally on the electric leads (21, 22, 23, 24) of said flexible connection (2),
   said digital control data are transmitted serially in the form of words on exactly one of said electric leads (21, 22, 23, 24).

17. The device of one of claims 1 to 16, said instrument (100) including an electric accessory, for example a lamp (29), of which one of the terminals is connected
   to one of the leads (21, 22, 23, 24) of the flexible connection (2) dedicated to this use, and/or
   to one of the phase conductors (21, 22, 23) of said motor (1), and/or
   to the neutral point between the phases of said motor (1), and/or
   to said digital electronic circuit (4) controlling its working.

18. The device of one of claims 1 to 17, wherein said electronic servo-control module (3) includes three power drivers (32, 33, 34) for feeding the three phases of said motor (1).

19. The device of claim 18, wherein said three power drivers (32, 33, 34) are analog drivers supplying three analog signals (83, 84, 85) phase-shifted by 120° and of variable frequency.

20. The device of one of claims 1 to 19, wherein said electronic servo-control module (3) controls the rotation speed of said motor (1) and/or the torque of said motor (1).

21. The device of one of claims 1 to 20, wherein said servo-control module (3) performs a correction of the set-point value to take into account the positioning errors of the Hall sensors (H1, H2) determined during calibration of the device.

22. The device one of claims 1 to 21, wherein said servo-control module (3) performs a correction of the set-point value to take into account the rotor's rotation during measurement and computation and/or to compensate the pursuit angle between the stator field rotated and the field of the rotor (10).

23. The device of claim 22, wherein the correction performed for compensating said pursuit angle is proportional to the voltage applied on said windings.

**24.** The device of claim 1 to 23, wherein said servo-control module (3) includes a processor controlled by a program for controlling the operation of said instrument (100), said program allowing to define the machining cycle with said tool (100), e.g. machining cycles comprising several back and forth wit said tool (100) for breaking chips.

**25.** The device of claim 1 to 24, wherein a shield is provided between the stator windings of said motor (1) and said digital electronic circuit (4).

**26.** The device of claim 1 to 25, wherein said at least one sensor (H1, H2) is lodged in a recess in the stator sheets of said motor (1).

**27.** The device of claim 1 to 26, wherein said at least one sensor (H1, H2) is lodged in a recess between the rotor (10) and the stator.

**28.** The device of claim 1 to 27, wherein said digital electronic circuit (4) is mounted on a printed circuit board perpendicular to the rotation axis of said motor (10), preferably at least one sensor (H1, H2) being mounted onto said printed circuit board.

**29.** The device of claim 1 to 28, wherein said instrument (100) includes a CCD sensor supplying data processed by said digital circuit (4) and transmitted to said electronic servo-control module (3).

Figure 1

Figure 2

Figure 3

DAP

Figure 4

H1  7  71  72  73

100

10

H2

Figure 5

Figure 6

Figure 7a

Figure 7b

Figure 7c

Figure 7d

Figure 8

Figure 9

Figure 10

HEADER

D0-D3 DATAs WORD

D4-D7 DATAs WORD

D8-D11 DATAs WORD

D12-D15 DATAs WORD

MSG n

MSG n+1

MSG n+2

MSG n+3

MSG n+4

Message auxiliaire complet

## Figure 11

Générateur
dent de scie
( 150kHz )

A
B
A>B

Udc

OUT

Consigne

A
B
k(A-B)

## Figure 12a

83
84
85

32
33
34

86

1

## Figure 12b

Figure 13

Figure 14

Figure 15

Figure 16

Figure 17

Figure 18

Figure 19

EP 1 670 377 B1

**EP 1 670 377 B1**

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 688539 A
- US 5543695 A
- EP 1228737 A
- EP 1302173 A
- WO 0105023 A
- WO 0145248 A
- EP 1109301 A
- WO 0004631 A
- WO 9814129 A